# EUROPEAN PATENT APPLICATION

(11) **EP 1 813 268 A1**
(43) Date of publication of application: **01.08.2007**
(21) Application number: 06012347.8
(22) Date of filing: 15.06.2006
(51) Int. Cl.: A61K 9/16, A61K 9/51

(54) **Method for preparing polymeric matrices by radiation in the presence of sensitive pharmaceutical compounds**

(71) Applicant: DSM IP Assets B.V., 6411 TE Heerlen (NL)
(72) Inventor: The designation of the inventor has not yet been filed
(74) Representative: Habets, Winand

(57) **Abstract**

The present invention relates to a method for the preparation of a drug loaded matrix with a pharmaceutical compound by polymerizing a polymerisation mixture by radiation in the presence of at least one sensitive pharmaceutical compound, wherein the polymerisation mixture comprises
a) at least one polymerisable compound
b) optionally at least one photoinitiator
c) optionally at least one additive
which are chosen such that the sensitive pharmaceutical compound is degraded less than 2%, or which are chosen such that the sensitive pharmaceutical compound is protected against degradation such that the degradation is about 50% or less when compared to radiation with a constituent that produce radicals.

## Description

The present invention relates to a method for preparing a polymeric matrix in the presence of pharmaceutical compounds. Specifically, the present invention relates to such a method with pharmaceutical compounds that are sensitive to reactive radical intermediates during polymerisation.

ln recent years, slow release preparations of drugs have become increasingly popular.This is driven by a number of benefits. The pharmaceutical compound generally is delivered in a more controlled manner, leading often to lower dosages than would be needed via systemic administration thereby minimising a number of unwanted side effects. Furthermore, the use of prolonged and controlled drug releasing matrices provides significant benefits in terms of health care cost by reducing the need for continual administration by health care workers. Also, slow release preparations offer the possibility of targeted drug release, for example if a such preparation is brought in the body at a specific place which is in need of the drug. The site of implantation provides a method of achieving targeted drug delivery since release of the pharmaceutical compound is often localised. Since the mobility of these implants is restricted it provides a manner of targeted drug delivery already. ln yet another application, matrices are prepared, which matrices are used for renewal of blood vessel, nerves, bones, muscular or other tissue. The renewed tissue may replace the matrix, or the matrix may stay in the body. It is preferred, that the matrix is loaded with one or more of pharmaceutical and or biologically active products.

Generally, the drugs are absorbed in a matrix, like a polymer or gel, after such polymer or gel has been made. See for example Kim et al. Phar. Res 9; 283-289 (1992). Such a system has some intrinsic disadvantages; the loading of the drug is strongly dependent on the molecular weight of the drug, polarity of the drug and the molecular weight and polarity of the matrix. Often the drug has to be dissolved in a solvent in order to improve the loading of the already formed matrix, as described by Gref et al. Science,1994, 263, 1600-1602. However, solvents or subsequent steps to remove the solvent can denature the drug, e.g. if the drug is protein based. This has lead to number of ways to enhance the drug loading of polymer gels as disclosed e.g. in WO96/02239 and WO95/15748.

A potentially better preparation can be made where the polymerisation to obtain the polymer to form a matrix is carried out in the presence of the drug, with the effect that the drug is *in situ* encapsulated. The polymerisation preferably is carried out with UV or Visible light induced radical polymerisation. The radical reaction that occurs during radiation initiated polymerisation or indeed any radical polymerisation can result in reactions between the radicals and the pharmaceutical compound. This is not only a disadvantage because valuable pharmaceutical compound is lost, but also because the resultant by-products may be damaging to health or cause unwanted side effects.

WO 2005/070467 describes a method to protect sensitive molecules from a photopolymerising environment. The reference suggests to apply to bioactive molecules an insoluble material that undergoes a solid-gel transition at body temperature. This has as a disadvantage that an additional step is necessary, and that yet other components are used, which need careful control for biocompatability and influence on drug release behaviour.

It is an object of the invention to provide a method for the preparation of a drug loaded matrix by polymerising a polymerisation mixture to form a matrix by radiation in the presence of a sensitive pharmaceutical compound wherein said pharmaceutical compound exhibits virtually no decomposition or chemical change .

It is a further object of the invention to prepare a drug loaded matrix with a sensitive pharmaceutical compound, wherein the pharmaceutical compound has retained substantially its activity.

This and other useful results are obtained in a method for the preparation of a matrix with a pharmaceutical compound by polymerizing a polymerisation mixture by radiation in the presence of a sensitive pharmaceutical compound, wherein the polymerisation mixture comprises
a) at least one polymerisable compound
b) optionally at least one photoinitiator
c) optionally at least one additive
which are chosen such that the pharmaceutical compound is degraded less than 2%

Another embodiment of the invention relates to a method for the preparation of a drug loaded matrix by polymerizing a polymerisation mixture by radiation in the presence of at least one sensitive pharmaceutical compound, wherein the polymerisation mixture comprises
a) at least one polymerisable compound
b) optionally at least one photoinitiator
c) optionally at least one additive
which are chosen such that the sensitive pharmaceutical compound is protected against degradation such that the degradation is about 50% or less when compared to radiation without constituents (a) and (c).

Pharmaceutical compounds may be affected by UV light, visible light, electron beam or gamma radiation, or by radicals or ions e.g. generated by photoinitiating systems. If the pharmaceutical compounds are not affected by the photopolymerization system in any way, these compounds can be used in the preparation of a matrix with *in-situ* loading of the pharmaceutical compound without any problem with respect to degradation, as these pharmaceutical compounds are neither sensitive to the light nor to the photopolymerisation system. Whether a pharmaceutical compound (hereinafter also named API, active pharmaceutical ingredient, or drug) is affected is not easily predicted.

Some guidelines have been proposed to test the UV and light sensitivity of pharmaceutical compounds. However, the ICH testing guidelines are designed to test long term, storage related light stability and are not suitable for solving the current problem. In the ICH testing guidelines, the light intensities involved are low, but exposure is long (over months or years). This results in light doses in the hundreds to thousands of J/cm². In contrast, exposure in the case photopolymerisation with drugs *in situ* involves short times (subsecond to minutes) and typically high light intensities. For example, in the case of photopolymerisation, matrices can be made by using UV or Visible radiation with a dose of about 0.2 or 3 J/cm². Sometimes, grafting reaction may use up to 10 J/cm² or more. The present inventors have developed a way which allows reliable testing.

A test method developed by the inventors requires testing the API at 2 J/cm² and a forced radiation test at 12 J/cm². Whether API's are affected by this level of irradiation can be measured in a number of ways. The inventors found most suitable methods as described below.

Unexpectedly, the inventors have furthermore found that such sensitive APl's can be protected by other components in the photopolymerisation system. Without wanting to be bound by theory, it now seems, in several of the drugs tested, that these other components have functional groups that effectively compete with the drug for radicals thereby protecting them. Suitable examples of functional groups present in these additives include, but are not limited to, unsaturated groups, strained rings, tertiary amines, thiols, organohalogens and other reactive radical groups that are able to interfere with free radical polymerisation via initiation, propagation chain transfer or termination reactions. When a functional group has been determined to be effective in protection, it is possible to use oligomers, and (other) monofunctional or polyfunctional reactive diluents with that functional group in the ultimate polymerization mixture to achieve protection of the API.

The API's - if attacked by radicals - will show a failure that generally can be attributed to bond scission, addition, grafting or hydrogen abstraction. Addition is a reaction with an ethylenically unsaturated double bond; grafting is a reaction to an atom not at a C=C double bond, and hydrogen abstraction can lead to grafting, but also to elimination of groups (bond scission) from the API. In order to protect an API, if the failure mode is addition, a compound which is able to undergo radical addition reactions e.g. a compound furnished with a suitable ethylenically unsaturated double bond will be effective. ln case of grafting or scission or hydrogen abstraction, it is possible to use compounds having ethylenically unsaturated groups, but one can also use chain transfer or terminating agents to protect the API. Examples of chain transfer agents include compounds comprising for example amines or thiols. Terminating agents generally are stabilisers, or compounds that interfere with radical reactions such as phenothiazine, stable radical compounds and those from the quinone family (e.g. Irganox 1035. hydroquinone, methylhydroquinone).

As used herein, the term sensitive pharmaceutical compounds is to be interpreted as compounds that degrade for more than 5 % in an environment with radical generation at 12 J/cm² irradiation. More in particular, the pharmaceutical can be sensitive during polymerisation that can be initiated with UV or visible light (200-700 nm) or with electron beam or gamma radiation, and without or up to 10 % radical generating species (initiator) present. If an initiator is present, it is preferably about 1-10 wt % of a photoinitiator. In addition to photoinitiators, the system may comprise also a thermal initiator (for example peroxide and/or redox initiators). Preferably a radiation sensitive (photo)initiator is used, for example a Norrish Type I or Type II initiator. It is possible to work in the absence of these radical generating species where electron beam or gamma radiation is used or in the case where unsaturated compounds (one compound whose double bond is electron rich and another compound whose double bond is electron poor) react with each other via a donor- acceptor complex to form a polymer.

Potential degradation can be measured by dissolving 100 ppm (0.1 g/l) pharmaceutical compound in a suitable solvent in a quartz, glass or HPLC vessel. One series of vessels is used as such. In a second series of vessels, the one or more initiators, preferably those used in the polymerization mixture, are added in the same amount as the pharmaceutical compound. Next, the vessels are subjected to irradiation at 2 and 12 J/cm². As an example, UV radiation can be performed with a lab UV source Bluepoint 2, equipped with a D-bulb D65 and directed onto the sample by a UV light guide. The output from the guide is 200 mW/cm² (200 mJ/cm² per second) as measured with a Solascope 1 UV spectrometer. A dose of 2 and 12 J/cm² was achieved by irradiating 10 and 60 seconds, respectively. The choice of irradiation system could be governed by the applications or processing considerations that typically influence hardware use in a laboratory, hospital or manufacturing environment. For example where the irradiation is performed in vivo for dental cements and adhesives,intraocular lenses, surgical sealants or barriers the preferred light soucre is often visible light. Thus for these applications a visible light source would be used instead of UV.

The recovery could be analysed by HPLC. In order to reliably measure the drug concentration before and after exposure to light, a reversed phase HPLC method was developed, to chromatographically separate the drug from the photoinitiator and polymerising species (like acrylates). A Hewlett Packard 1090 HPLC apparatus equipped with a X Terra RP 18 (3.5 µm) column was used to separate the drug from the initiator and (meth)acrylate monomer and establish the drug concentration in solution. Acetonitrile and a 10 mM phosphoric acid in water solution were used as the eluents. In this screening set up the monomers are chosen such that the resulting polymer is not chemically crosslinked to form a polymer network, since this may entrap the drugs and render subsequent analysis difficult. UV detection at about 250 nm was used. A linear calibration curve (drug peak area vs. drug concentration) was obtained for the drugs in the range of concentrations used. UV spectra of the drugs before and after UV irradiation (at both 2 and 12 J/cm²) had identical shapes, meaning that the HPLC method could be used to quantitatively measure changes in drug concentrations. Any degradation would be calculated as: minus the percentage of recovered API.

A more detailed assessment is possible and useful to check whether degradation has occurred and if side products are formed. Preferably, UV spectra of eluted compounds are measured, e.g. with a Photo Diode Array. Further, preferably MS spectra are measured from the eluted products, to look for any degradation products. A suitable method for example comprises FIA-PDA-ESI-MS as a fast screening method. However, it is even more preferred to use HPLC-PDA-ESI-MS (FIA: Flow Injection Analysis; HPLC: High Performance Liquid Chromatography; PDA: Photo Diode Array detection; ESI: electro spray ionization; MS: mass spectrometry). The applied reversed phase method is not suitable to separate enantiomers. Many active pharmaceutical ingredients are chiral molecules and could show light or radical sensitivity by isomerization or racemization. These processes also lead to reduction of pharmaceutical activity or even toxic components. An additional analytical tool is required to detect these molecular changes. In case the drug is considered chemically stable with HPLC-PDA-ESI-MS, a more detailed analysis on chirality can be performed by applying optical rotation measurements, for example with FIA-ALP (ALP: advanced laser polarimetry) or more preferably with HPLC-PDA-ALP.

PDA (UV spectrometry) shows the overall absorption, which gives information with respect to the potential of light absorption from the UV-source and any changes in the light absorption spectrum suggest chemical changes.

ESI-MS (mass spectrometry) provides fingerprint mass spectrum and will reveal whether fragmentation or addition or other chemical changes occur.

With FIA-APL, (polarimetry) any change in optical rotation can be measured in order to detect change in chiral activity.

As a solvent any solvent in which the pharmaceutical compound dissolves may be suitable. Examples of suitable solvents include but are not limited to, water, ethanol, iso-propanol, tetrahydrofuran, hexane, cyclohexane, toluene, DMF, DMSO, NMP, acetone, methyl ethyl ketone, and halogenated solvents like dichlorormethane, chloroform, and mixtures of any of these solvents. It is preferred to use a solution, as that allows one exaggerate potential degradation. Alternatively, one or more constituents including the drug may be dispersed (e.g. as oil in water emulsion or water-in-oil emulsions, or dispersion).

The method of the present invention is in particular suitable for making a matrix comprising a sensitive pharmaceutical compound.

A number of pharmaceutical compounds are shown to be radical sensitive in the current testing method, such as Terazosin®, Prazosin®, Rapamycin®, Chloramphenicol® and Amiodarone®. Others can be tested, as described above.

With a selection of suitable components, the degradation can be reduced by about 50% or more, or even about 70% or more.

In one embodiment, the degradation at 12 J/cm² is about 4% or less, preferably about 3% or less, and even more preferably about 2% or less.

In another embodiment, the degradation at 2 J/cm² is about 1% or less, preferably about 0.5% or less.

The matrix can be made from radically polymerisable components that are monomeric or polymeric and possess radically reactive and /or unsaturated bonds. The matrix may be formed into gels or as coatings, films, adhesives, laminates, micro and nanospheres, vesicles, liposomes, polymerosomes, moulded articles e.g. lumen bearing or filled tubes, fibres, weaves, meshes and a number of 3 dimensional monoliths of various complexity that are available in polymer processing including rapid prototyping and other layer by layer manufacturing processes.

In one embodiment the drug loaded matrix is used for drug delivery. In another embodiment, the drug loaded is used for tissue engineering. In another embodiment, the drug loaded matrix is used for diagnostic purposes. In another embodiment, the drug loaded matrix is used for detoxification or substance removal. ln another embodiment the matrix serves as a substrate or scaffold for cellular growth, differentiation and proliferation.

In one embodiment, the drug loaded matrix is made in a laboratory or manufacturing plant. This can be an advantage in particular in making drug loaded matrices for drug delivery or for diagnostic purposes.

In another embodiment, the drug loaded matrix is made *in vivo,* when treating a patient or animal as has been described e.g. by Langer et al Proc. Nat. Acad. Sci. 96,3104 (1999) and US 6,602,975.

In one embodiment, the drug loaded matrix is self-contained. It either can be used as such, or can be fixed to a carrier afterwards.

In another embodiment, the drug loaded matrix is prepared on a carrier. The carrier can be biostable or biodegradable.

Examples of carriers include, but are not limited to, films, tubes or functionally shaped medical devices where the shape of the device is designed to aid its function either for implantation or insertion into the human or animal body or its function upon insertion in the body, e.g. hydrogels, catheters, stents, stent grafts, tubes, sponges, plasters and adhesive gels.

In one embodiment, the matrix is degradable in-vivo.

In another embodiment, the matrix is non-degradable in-vivo.

Drug loaded matrices, optionally on a carrier, preferably are sterilized or made or processed in a sterile environment.

The pharmaceutical compound can be any compound useful for treating human or animals or for treating side effects that are a consequence of implantations or invasion in the body. Particularly suitable are pharmaceutical compounds that are effective at targeted places or in slow release systems. The drug loaded matrix may comprise one or more pharmaceutically or biologically active compounds, at least one of these is a sensitive pharmaceutical compound. Examples of suitable pharmaceutical compounds that may or may not be sensitive to radicals include, but are not limited to, growth factors, anti-coagulation factors for blood, anti-inflammation medicines, anticancer agents, antihypertensive, antithrombogenic agents, analgesic, calcifying agent., neuron blocking agents, neurotransmitters, vaccines, hormones, antispasmodic agents, antimigraine agents, muscle relaxants, diuretics, uterine, anaesthetics, antibiotics, antiviral agents, cytokines, cardiovascular drugs, histamines and antihistamines, immunosupressants, vitamins, imaging or contrast agents( Xray, MRI or ultrasound), therapeutic proteins or peptides.

The matrix is prepared by polymerisation of a mixture comprising at least one of monomer, oligomer or polymer. In one embodiment of the invention, the matrix comprises linear polymers that may be soluble or insoluble. In case the polymer is soluble, it is preferred that the dissolution rate is low to allow slow release of the pharmaceutical compound. ln another embodiment of the invention, the matrix consists of a polymer network.

The polymer network could be rendered soluble upon degradation.

The physical characteristics of the matrix are largely dependent on the chemical compounds used and the drug to be contained in the matrix. The matrix can have physical characteristics as necessary for its use. The matrix can be e.g. hard or very soft and compliant. Hard and stiff drug loaded matrices can be made as bone replacement or cement in bone repair or any compressive load bearing part of the body. Soft but strong drug loaded matrices can be useful in areas of the body where soft tissue exists and optionally where patient comfort and compliance is an issue, like in blood-vessel implants, tendons , muscles, or any tensile load bearing parts of the body. The required characteristics can be formed by the suitable chemical compounds.

Polymerisation of the polymerization mixture to obtain the matrix may advantageously be accomplished by using radiation. Radiation initiated polymerisation is preferred over heat initiated polymerisation, because temperature can damage heat sensitive pharmaceutical compounds such as proteins. However heat initiated polymerization that involves radical reactions could also be considered. The radiation can be any electromagnetic radiation from UV, Visible, electron beam or gamma irradiation. It is preferred to have irradiation energy chosen as low as possible, but while still achieving decent cure rates and substantial cure of the polymerization compounds as to obtain a matrix that can effectively hold the pharmaceutical compound.

Generally, the irradiation energy will be about 0.2 J/cm² or higher, preferably about 0.5 J/cm² or higher, such as for example about 0.8 J/cm² or higher, such as for example about 1 or about 2 J/cm². Generally, the irradiation energy will be about 5 J/cm² or lower, preferably, about 3 J/cm² or lower.

The polymerisation is carried out with the pharmaceutical compound present. This is an advantage, as the compound is much more evenly distributed either as a solution or as a dispersion, and the drug loaded matrix can be made in one step. It also gives the option to make multi-layer systems with different loadings of pharmaceutical compound in the different layers. It furthermore allows in-vivo applications. The pharmaceutical compound should be present during the formation of the matrix. In case the polymerisation would take place in a more than one step, e.g. with a first step only forming small molecules, the pharmaceutical compound needs only to be present in the final step where the ultimate matrix is formed.

The polymerization mixture can comprise a number of compounds suitable to form a matrix upon polymerisation.

In case a cross-linked (chemical) network is to be obtained, generally, the mixture comprises at least one ethylenically unsaturated compound which is multifunctional. A wide variety of compounds may be suitable.

In case linear polymers are used to make a matrix, generally monofunctional compounds are used. Hereinafter, this type of matrix is also named a physical network.

Polymerisable compounds generally comprise reactive diluents and reactive oligomers and optionally non reactive polymers or oligomers that - when polymerised - would result in the formation of a physical or chemical network, that may be of a semi interpenetrating nature. The oligomers often are used to facilitate the formation of a chemical network, as the oligomers often are multi-functional. The reactive diluents can be monofunctional or multifunctional. Thus, depending on the required characteristics of the ultimate matrix, the polymerization composition comprises oligomers and diluents and optionally non reactive oligomers and monomers to achieve required physical properties. The properties include modulus, degradability, flexibility, surface slip characteristics, and stress - strain behaviour. In polymerizing the polymerization mixture, polymers are formed that may be linear, branched, or comb-like polymers which may have mainly physical entanglements or may be chemically crosslinked to for a network.

Generally, oligomers will be bio-degradable materials or non bio-degradable materials and have a molecular weight of about 400 or higher, preferably about 800 or higher. Generally, the molecular weight will be about 20 000 or lower, preferably about 10,000 or lower.

Generally, the oligomers used to make a chemical (three dimensional) network will have an average functionality of about 1.3 or higher, preferably about 1.8 or higher. Generally, the functionality will be about 30 or lower, preferably about 10 or lower, and more preferably about 8 or lower.

Oligomers that are used to make a physical network will have an average functionality of about one, or lower.

As used herein, functionality is the capability to form two bonds effective to form a linear polymer. E.g. a mono-functional acrylate in radical polymerisation is capable to form two bonds. As another example, thiols can react with vinyl-ethers in a step growth polymerisation. In this case, monofunctional means that a compound has on-average two thiol or two vinyl groups, or one vinyl and one thiol, because the thiol adds to a vinyl group forming only one bond that is effective in forming a polymer.

Examples of oligomers include, but are not limited to: polyether, polyester, polyurethanes, polyamides, polypeptides, polyanhydride, polyorthoesthers, polythioesters, polyhydrocarbons, polycarbonates, polyureas, polysulphones, and poly acrylamide, polylactide, polyglycolide, polydioxanone, poly(lactide-co-glycolide), poly(glycolide-copolydioxanone), polyanhydrides, poly (glycolide-cotrimethylene carbonate), poly-hydroxyalkanoates, and poly(glycolide-co-caprolactone). Suitable examples include, but are not limited to: polyethylene-glycol with a Mw of 8000, 6000, 600, 3400, or 4600, poly-acrylamide-hydroxyethylacrylate copolymers, polyorthoesthers and poly[bis(p-carboxyphenoxy)propane-co-sebasic acid)], polyterephthalic acid polysebacic acid and the like.

Oligomers can also be based on natural occurring compounds such as dextran, cellulose or gelatin.

The oligomers generally comprise reactive groups such as for example, acrylate, methacrylate, fumarate, maleate, itaconate or allylmalonate, vinyl, propenyl and butenyl ethers, allyl ethers and allyl esters, vinyl amines and vinyl amides, styryl groups, maleimides, itaconimides, cinammates, thiols or oxazolines. Oligomers are commercially available, or can be synthesised. For example, these reactive groups can be introduced directly, or via step-reactions. Acrylic polymers with hydroxy side-chains can be functionalized with acrylic acid-chloride, acrylate group containing isocyanate compounds, or epichlorhydrine. The epoxygroup can thereafter be functionalized with acrylic acid or amine groups. Acid functional oligomers, such as polyacid-anhydrides can be functionalized with epoxy-propylmethacrylate.

Examples of compounds suitable to make a linear polymer for a (physical) network include polyethyleneglycol-monoacrylate, polylactic-acid monoacrylate, vinyl pyrollidone, styryl compounds, vinylimidazole and di-thiol and diene-functional oligomers or lower molecular weight compounds.

ln one embodiment of the invention, the photopolymerization mixture further comprises at least one reactive diluent. The molecular weight of the reactive diluent generally will be about 700 or lower, preferably about 400 or lower. The reactive diluent will have radically reactive functionalities between 1 and 8.

Examples of suitable diluents include, but are not limited to, hydroxyethyl(meth)acrylate, Hydroxybutyl(meth)acrylate, N-vinylpyrrolidone, acrylamide, triethyleneglycol (meth)acrylate, diethyleneglycol-di(meth)acrylate, hexane dioldimethacrylate, isobornyl(meth)acrylate, iso-decyl(meth)acrylate, ethoxylated lauryl (meth)acrylate, 2-ethyl-hexyl(meth)acrylate, N-vinylcaprolactam

Photoinitiators used in the present invention generally are either Norrish type I or type II photoinitiators.

Norrish type I photoinitiators are cleaved by UV and/or Visible light to generate radicals, which initiate the radical polymerisation reaction. Examples of Norrish type I photoinitiators include, but are not limited to: dimethoxy acetophenone, aminoalkylphenones, benzyl ketals, hydroxyalkyl phenones and acylphosphineoxides.

Norrish type II photoinitiators generate radicals through reaction with a proton-donor, generally a dimethylamine compound. Examples of Norrish type II photoinitiators include, but are not limited to, benzophenone, xanthones, thioxanthones, coumarins, aromatic 1,2 diketones, phenylglyoxylates, camphorquinones and Eosin Y combined with dimethyl amines, such as dimethylethanol amine.

Additives can be useful to influence properties. For example, soluble polymers can be used to increase viscosity, siloxanes can be used to make a matrix more water-repellent. In one embodiment of the present invention, one or more of these additives are reactive to the radicals and may be reactive double bonds, thiols or radical scavengers eg TEMPO. lf reactive to radicals, the additives may serve to protect the pharmaceutical compound.

The invention will be elucidated by the following inventions, without being limited thereto.

### EXAMPLES

### Example I: Methods

### UV irradiation:

UV irradiation of drug-containing solutions (see below) was performed with a lab UV source Dr. Hoenle Bluepoint 2, equipped with a D-bulb, directed onto the (open) HPLC vials (2 ml) by a UV light guide (200 mW/cm²; 200 mJ/cm² per second as measured with Solascope 1 UV emission spectrometer). The (unstirred) solutions were exposed to 2 J/cm² ("low" UV dose) or 12 J/cm² ("high" UV dose). After exposure to UV light the vials were shaken to homogenize the samples.

### Phase (i) testing:

5 mg drug (amiodarone®, prazosin®, chloramphenicol® or terazosin®) was dissolved in 50 ml water/ethanol (1/1 v/v).

3 HPLC vials were filled with 1 ml of this drug solution. One vial was not exposed to UV light (reference); one vial was exposed to 2 J/cm² UV light (10 seconds exposure to Bluepoint 2 light guide); one vial was exposed to 12 J/cm² UV light (60 seconds exposure to Bluepoint 2 light guide).

### Phase (ii) testing:

5 mg drug and 5 mg photoinitiator (lrgacure 2959 or 819) were dissolved in 50 ml water/ethanol (1/1). Weight ratio drug/photoinitiator = 1.

3 HPLC vials were filled with 1 ml of this solution. One vial was not exposed to UV light (reference); one vial was exposed to 2 J/cm² UV light (10 seconds exposure to Bluepoint 2 light guide); one vial was exposed to 12 J/cm² UV light.

### Phase (iii) testing:

5 mg drug, 5 mg photoinitiator and 0.5 g (meth)acrylate were dissolved in 50 ml water/ethanol (1/1) and combined with a polymerisable compound. HEA or HEMA: both are soluble in water/ethanol; IDA (isodecyl acrylate) does not dissolve in water/ethanol (1/1) and forms an emulsion. Weight ratio drug/photoinitiator = 1; ratio acrylate/drug = 100. For testing purposes, the polymerisable compound is a monofunctional reactive compound. Polymerising such a compound should yield linear polymers only. This would preclude that the pharmaceutical compound would be physically entrapped in a polymer network.

3 HPLC vials were filled with 1 ml of this solution. One vial was not exposed to UV light (reference); one vial was exposed to 2 J/cm² UV light (10 seconds exposure to Bluepoint 2 light guide); one vial was exposed to 12 J/cm² UV light.

Phase (i), (ii) and (iii) experiments were done in 5 fold. Results are the average of 5 (independent) measurements.

### Analysis:

High pressure liquid chromatography (HPLC) was the analytical technique used to measure and compare the drug concentration before and after exposure to UV light.

HPLC was used to separate the drug from the photointiator and (meth)acrylate monomer and establish the drug concentration in solution.

All drug concentration measurement were done by liquid chromatography. The HLPC system (HP 1090 Liquid Chromatograph) consisted of the following components: DR5 pump, diode array detector (DAD), built-in autosampler, and ChemStation software, version Rev. A. 08.03 (Agilent Technologies). A C18 analytical column 150 x 4.6 mm (XTerra RP 18, Waters) with a mean particle size of 3.5 µm was used at 40.0 °C. Acetonitril and a 10 mM phosphoric acid/water solution were used as the eluents. A flow rate of 1.5 ml/min and injection volumes of 5 µl were used during the analysis.

UV detection was used for all drugs. A linear calibration curve (drug peak area vs. drug concentration) was obtained for the drugs in the range of concentrations used. UV spectra of the drugs before and after UV irradiation (at both doses: 2 and 12 J/cm²) had identical shapes, meaning that the HPLC method could be used to quantitatively measure changes in drug concentrations.

For every drug a HPLC method was developed to chromatographically separate the drug from the photoinitiator and monomer (acrylate-type).

### Eluent gradients:

For prazosin® the following cycle was developed: during one gradient cycle of 14 min, the mobile phase was changed from 17 to 80 % of mobile phase Acetonitrile over a period of 8 min, kept at 80 % for 2 min and thereafter, lowered to 17 % in 4 min, where it was kept until the next sample was injected. The detection was done at 250 and 340 nm.

For terazosin®, during one gradient cycle of 14 min, the mobile phase was changed from 10 to 80 % of mobile phase Acetonitrile over a period of 8 min, kept at 80 % for 2 min and thereafter, lowered to 10 % in 4 min, where it was kept until the next sample was injected. The detection was done at 250 and 340 nm.

For amiodarone® a slightly modified HPLC method was developed: isocratic HPLC runs taking 5 minutes were carried out using a 50/50 ACN/10mM H₃PO₄ solvent mixture as the mobile phase. UV detection was done at (230 and) 254 nm.

For chloramphenicol®, during one gradient cycle of 10 min, the mobile phase was changed from 30 to 70 % of mobile phase acetonitrile over a period of 5 min, kept at 70 % for 1 min and thereafter, changed to 90 % of mobile phase acetonitrile over a period of 0.1 min, again kept at 90 % for 1 min, and lowered to 30 % in 0.1 min, where it was kept until the next sample was injected. A flow rate of 1.5 ml/min and an injection volume of 5 µl were used during the analysis. The detection was done at 200 and 280 nm.

### Example II

The required amount of Prazosin® was dissolved in 50% water, 50% ethanol to make a 100 ppm solution, and put in 1 ml HPLC vessels. Further vessels were used together with 100 ppm lrgacure 2959 photoinitiator, and with photoinitiator and polymerisation components, as shown in the following table.

| Composition | Irradiation 2 J/cm² | Irradiation 12 J/cm² |
|---|---|---|
| Prazosin® | 99 % recovery | 98 % recovery |
| Prazosin® with Irg 2959 | 97 % recovery | 89 % recovery |
| Prazosin® with Irg 2959 and Hydroxyethylacrylate | 97 % recovery | 92.5 % recovery |
| Prazosin® with Irg 2959 and hydroxyethylmethacrylate | 98.5 % recovery | 97 % recovery |

### Example III

In a separate experiment, the required amount of Prazosin® was dissolved in 50% water, 50% ethanol to make a 100 ppm solution, and put in quartz HPLC 1 ml vessels. Further vessels were used together with 100 ppm Irgacure 2959 photoinitiator, and with photoinitiator and polymerisation components, as shown in the following table. DMEA is di-methyl ethanol amine.

| Composition | Irradiation 2 J/cm² | Irradiation 12 J/cm² |
|---|---|---|
| Prazosin® | >99% recovery | 98.5% recovery |
| Prazosin® with lrg 2959 | 98% recovery | 90% recovery |
| Prazosin® with lrg 2959 and Hydroxyethylacrylate | 98% recovery | 92.5% recovery |
| Prazosin® with lrg 2959 and hydroxyethylacrylate and DMEA | 98% recovery | 96% recovery |
| Prazosin® with Irg 2959 and DMEA | >99% recovery | 97% recovery |

This example shows that the experiments are well reproducible within error margin (± 1%). The error margin may be smaller when more experiments are done to average out errors.

### Example IV

The required amount of Chloroamphenicol was dissolved in 50% water 50% ethanol to obtain a 100 ppm solution, which was put into a 1 ml HPLC vessels. Further vessels were used with lrgacure 2959, and a mixture of this with poly(propyleneglycol)mono-acrylate, as shown in the following table.

| Composition | lrradiation 2 J/cm² | Irradiation 12 J/cm² |
|---|---|---|
| Chloroamphenicol | 100 % recovery | 100 % recovery |
| Chloroamphenicol with Irg 2959 | 100 % recovery | 90 % recovery |
| Chloroamphenicol with Irg 2959 and poly(propyleneglycol)acrylate | 100 % recovery | 98 % recovery |

### Example V

The required amount of Amiodarone® was dissolved in 50% water 50% ethanol to obtain a 100 ppm solution, which was put into a 1 ml HPLC vessels. Further vessels were used with lrgacure 819, and hydroxyethylacrylate, and a mixture of these, as shown in the following table.

| Composition | Irradiation 2 J/cm² | Irradiation 12 J/cm² |
|---|---|---|
| Amiodarone® | 99.5% recovery | 93 % recovery |
| Amiodarone® with lrg 819 | 87 % recovery | 56 % recovery |
| Amiodarone® with lrg 819 +and hydroxyethylacrylate | 94 % recovery | 78 % recovery |

### Example VI

The required amount of Terazosin® was dissolved in 50% water 50% ethanol to obtain a 100 ppm solution, which was put into a 1 ml HPLC vessels. Further vessels were used with lrgacure 2959. The assessment (iii) was this time performed as a bulk polymerization of hydroxyethylacrylate, with 2 % terazosin® and 1 % lrgacure 2959. Results are as shown in the following table.

| Composition | lrradiation 2 J/cm² | Irradiation 12 J/cm² |
|---|---|---|
| Terazosin® | 100 % recovery | 100 % recovery |
| Terazosin® with Irg 2959 | 98 % recovery | 89 % recovery |
| Terazosin® with Irg 2959 and hydroxyethylacrylate | 98 % recovery | 95 % recovery |

As is clear from these examples, the choice of appropriate constituents enables the man skilled in the art to find with easy and straightforward analysis techniques, with the knowledge provided in this specification, to find systems that allow in-situ polymerisation with sensitive pharmaceutical compounds to be protected against degradation to be useful in practise, or at least, to find ways to protect degradation for 50% or better. In the above experiments, which were set-up to preclude entrapping of pharmaceutical compound, degradation is 100% minus the percentage of recovery.

## Claims

1. Method for the preparation of a drug loaded matrix containing a pharmaceutical compound by polymerizing a polymerisation mixture by radiation in the presence of at least one sensitive pharmaceutical compound, wherein the polymerisation mixture comprises
a) at least one polymerisable compound
b) optionally at least one photoinitiator
c) optionally at least one additive
which are chosen such that the sensitive pharmaceutical compound is degraded less than 2%.

2. Method according to claim 1, wherein the pharmaceutical compound is degraded less than 1 % measured at the dose of curing said mixture.

3. Method for the preparation of a drug loaded matrix containing a pharmaceutical compound by polymerizing a polymerisation mixture by radiation in the presence of at least one sensitive pharmaceutical compound, wherein the polymerisation mixture comprises
a) at least one polymerisable compound
b) optionally at least one photoinitiator
c) optionally at least one additive
which are chosen such that the sensitive pharmaceutical compound is protected against degradation such that the degradation is about 50% or less when compared to radiation without constituents (a) and (c).

4. Method according to any one of claims 1-3, wherein the pharmaceutical compound degrades for more than 5% in presence of a photoinitiator when irradiated with UV or Visible light at 12 j/_{CM}².

5. Method according to any one of claims 1-4, wherein the polymerisation mixture comprises a photoinitiator and is cured by irradiation with UV-Visible light at about 0.2 J/cm² or higher, and about 3 J/cm² or lower.

6. Method according to anyone of claims 1-4, wherein curing is performed with electron beam or gamma radiation.

7. Method according to any one of claims 1-6, wherein the pharmaceutical compound is chosen from group consisting of growth factors, anti-coagulation factors for blood, anti-inflammation medicines, antithrombogenic agents, anticancer agents, antihypertensive, analgesic, calcifying agent, neuron blocking agents, neurotransmitters, vaccines, hormones, antispasmodic agents, antimigraine agents, muscle relaxants, diuretics, uterine, anaesthetics, antibiotics, antiviral agents, cytokines, cardiovascular drugs, histamines and antihistamines, immunosupressants, vitamins, therapeutic peptides and proteins imaging or contrast agents.

8. Method according to any one of claims 1-7, wherein the drug loaded matrix, optionally on a carrier has the form of a gel, coating, film, adhesive, laminate, micro or nanosphere, vesicle, liposome, polymerosome, moulded article e.g. lumen bearing tube, filled tube, fibre, weave, mesh, sponge or a 3 dimensional monolith.

9. Method according to any one of claims 1-8, wherein the drug loaded matrix comprising a matrix and pharmaceutical compound, the matrix being a linear, branched or crosslinked polymer network, which is prepared by polymerising reactive diluents and reactive oligomers.

10. Method according to any one of claims 9, wherein the matrix is degradable in-vivo.

11. Method according to any one of claims 9, wherein the matrix is non-degradable in-vivo.

12. Method according to any one of claims 1-11, wherein an oligomer is used for making the matrix, the oligomer being chosen from the group consisting of : polyether, polyester, polyurethanes, polyamides, polypeptides, polyanhydride, polyorthoesthers, polythioesters, polyhydrocarbons, polycarbonates, polyureas, polysulphones, and poly acrylamide, polylactide, polyglycolide, polydioxanone, poly(lactide-co-glycolide), poly(glycolide-copolydioxanone), polyanhydrides, poly (glycolide-cotrimethylene carbonate), polyhydroxyalkanoate and poly(glycolide-co-caprolactone).

13. Method according to any one of claims 1-12, wherein the polymerisable compound comprises an acrylate, methacrylate, vinylether, vinylesters. vinylamide or styryl group, fumarates, acetylenes, cinnamates, thiols.
